Europäisches Patentamt

European Patent Office

Office européen des brevets

(11) Publication number: **0 255 124**
**A2**

(12) **EUROPEAN PATENT APPLICATION**

(21) Application number: 87111005.2

(22) Date of filing: 29.07.87

(51) Int. Cl.4: **C12N 9/34** , C12N 1/20 , C12P 19/20 , C12M 1/40 , //C12N9/28,(C12N1/20,C12R1:1-45),(C12N9/34,C12R1:145)

(30) Priority: 29.07.86 JP 176722/86
08.08.86 JP 185129/86

(43) Date of publication of application:
03.02.88 Bulletin 88/05

(84) Designated Contracting States:
CH DE FR GB IT LI NL SE

(71) Applicant: HITACHI, LTD.
6, Kanda Surugadai 4-chome
Chiyoda-ku Tokyo 101(JP)

(72) Inventor: Haga, Ryooichi
11-3, Daiharacho-3-chome
Hitachi-shi(JP)
Inventor: Ishida, Masahiko
20-8, Hanayamacho-1-chome
Hitachi-shi(JP)
Inventor: Satoh, Masami née Tsuchiya
417-304, Namiki-4-chome Sakuramura
Niihari-gun Ibaraki-ken(JP)

(74) Representative: Strehl, Schübel-Hopf,
Groening, Schulz
Widenmayerstrasse 17 Postfach 22.03 45
D-8000 München 22(DE)

(54) Thermostable glucoamylase, a method for production of glucose using same and a plant for production thereof.

(57) The thermostable glucoamylase of the present invention is highly active even in acidic pH ranges at high temperatures of 70°C or above.

According to the process for production of glucose and production plant of the present invention, the liquefaction and saccharification of starch can be performed, without requiring either any cooling step or any neutralizing step with alkalis, because of the use of the thermostable glucoamylase of the present invention in combination with known acid-resistant and thermostable α-amylase.

EP 0 255 124 A2

## THERMOSTABLE GLUCOAMYLASE, A METHOD FOR PRODUCTION OF GLUCOSE USING SAME AND A PLANT FOR PRODUCTION THEREOF

FIELD OF THE INVENTION

The present invention relates to a thermostable glucoamylase, a method for saccharification of starch using the same and a plant for production thereof. More particularly, the present invention relates to an acid-resistant and thermostable glucoamylase and a method for production of glucose using the same as well as a plant for production thereof which requires neither cooling step nor neutralizing step.

DESCRIPTION OF THE RELATED ART

In order to produce sweeteners such as glucose, an isomerized glucoses, etc. from the starch, starch is firstly hydrolyzed by an $\alpha$-amylase to be liquefied to dextrin and then saccharified by glucoamylase, which is disclosed in U.S. Patent Nos. 4,578,352 and 4,6I3,570.

As such $\alpha$-amylases and glucoamylases play a leading role in the starch processing industry.

Conventional enzymes are stable only at ordinary temperature or below. For preventing the propagation of various undesirable bacteria and/or fungi and for increasing the reaction rate in a glucose production line, it is desired to conduct the reaction in a temperature range of 60°C or above. In addition, a starch slurry as a raw material shows acidity of pH 4 to 5. For this reason, it has been desired to develop enzymes having thermostability in acidic pH ranges.

However, $\alpha$-amylases currently used in an industrial scale have the optimum pH for the reaction in neutral pH ranges and its stable region is also in neutral pH ranges, while they have thermostability. Further these $\alpha$-amylases can exhibit their thermostability only in the presence of several mM calcium ions.

Therefore, the present inventors previously developed as an acid-resistant $\alpha$-amylase capable of liquefying starches a novel $\alpha$-amylase having excellent thermostability even at pH 4 (European Patent Publication No. 0I840I9), which enabled to the liquefaction of the starch slurry at it was in acidic pH ranges without requiring the addition of calcium agents. With regard to the glucoamylase used for the saccharification which is performed subsequent to the liquefaction step, however, any thermostable enzyme operable under acidic conditions showing a pH value of 4 to 5 has not been developed yet.

That is, glucoamylases currently employed in an industrial scale are produced from bacteria of the genus Rhizopus and the genus Aspergillus but both are enzymes used at ambient temperature so that rapid inactivation of the enzyme activity occurs at temperatures higher than 50°C (Handbook of Enzyme Application, page 62, published by Chijinkan Publishing Co., Ltd. I982). With respect to glucoamylases having excellent thermostability, there are known enzymes reported by V. Basaveswara Rao et al. - (Biochem. J., 193, 379 (I98I) or Katkocin et al. (U.S. Patent No. 4,536,477) but in any case, neutral conditions showing pH of approximately 6 are required to exhibit the thermostability. For this reason, an alkali should be added to the liquefied starch solution in the prior art saccharification step to neutralize the reaction system. As a result, in order to obtain the final products such as glucose, an isomerized syrups, etc., a neutralizing agent must be removed after the reaction, resulting in a marked increase of the burden on the desalting step using ion exchange resin. There are U.S. Patent Nos. 4,536,477, 4,600,693 and 4,284,722 as disclosing such invention.

SUMMARY OF THE INVENTION

An object of the present invention is to eliminate the foregoing problems in the prior art supra and to provide a novel glucoamylase usable under conditions of pH 5 or less at temperatures of 60 to 75°C in the production of glucose, etc. using starch as a raw material.

Another object of the present invention is to provide a thermophilic anaerobic bacterium capable of producing such a novel glucoamylase.

A further object of the present invention is to provide a method for production of glucose requiring neither cooling step nor neutralizing step by the use of a glucoamylase operable under acidic conditions showing pH 5 or less at high temperatures of 60 to 70°C in the production of glucoses using starch as a raw material and its production plant.

These and other objects and advantages of the present invention will be apparent from the following description.

A first aspect of the present invention relates to an acid-resistant and thermostable glucoamylase having the following physicochemical properties:

(I) Action optimum pH: 4 to 5

(2) Action optimum temperature: about 70°C

(3) Stable pH: 4.5 to 5.0

(4) A half life of enzyme activity at pH 4.5 and a temperature of 70°C is at least 6 hours.

(5) A half life of enzyme activity at pH 4.0 and a temperature of 70°C is at least 5.5 hours.

(6) Molecular weight: $3.8 \times 10^4$.

A second aspect of the present invention relates to a thermophilic anaerobic bacterium belonging to the genus Clostridium capable of producing the thermostable glucoamylase having the physicochemical properties described above.

A third aspect of the present invention relates to a method for production of glucose by saccharification of dextrin using a glucoamylase, which comprises reacting the raw dextrin at a temperatures of at least 70°C in acidic pH ranges substantially without adding metal ions to saccharify the dextrin.

A fourth aspect of the present invention relates to a method for production of glucose from starch by liquefying the starch using an $\alpha$-amylase and then saccharifying dextrin produced using glucoamylase, which comprises reacting the raw starch at a temperature of at least 70°C in acidic pH ranges substantially without adding metal ions to perform the liquefaction and saccharification of the raw starch.

A fifth aspect of the present invention relates to a method for production of glucose from starch by performing the liquefaction and saccharification by adding the $\alpha$-amylase and glucoamylase to the reaction system simultaneously, which comprises reacting the raw starch at a temperature of at least 70°C in acidic pH ranges substantially without adding metal ions.

A sixth aspect of the present invention relates to a plant for producing a glucose by liquefaction of a starch using an $\alpha$-amylase followed by saccharification using a glucoamylase, which comprises:

a liquefaction tank comprising a starch slurry making tank comprising a starch storage tank, a water storage tank and an $\alpha$-amylase storage tank, and a slurry heater; and,

a saccharification tank comprising a glucoamylase storage tank, having connected with said liquefaction tank.

## BRIEF DESCRIPTION OF THE DRAWINGS

Figure I is a characteristic graph showing the effect of pH on the glucoamylase activity of the present invention.

Figure 2 is a characteristic graph showing the effect of pH on the thermostability of the glucoamylase.

Figure 3 is a characteristic graph showing the effect of temperature on the glucoamylase activity of the present invention.

Figure 4 is a characteristic graph showing the thermostability of the glucoamylase.

Figure 5 is a glucoamylase activity elution pattern graph of an ion exchange liquid chromatography on diethylaminoethylated crosslinked agarose gel.

Figure 6 is a glucoamylase activity elution pattern graph of gel filtration on crosslinked dextran gel.

Figure 7 is a characteristic graph showing the effect of pH on the $\alpha$-amylase activity used in the present invention.

Figure 8 is a characteristic graph showing the effect of temperature on the $\alpha$-amylase.

Figure 9 is a characteristic graph showing the thermostability of the $\alpha$-amylase.

Figure I0 is a characteristic graph showing the improved thermostability of the $\alpha$-amylase by the addition of calcium.

Figure II is a flow chart showing an example and

Figure I2 is a flow chart showing another example, of the device for production of glucose according to the present invention.

## DETAILED DESCRIPTION OF THE INVENTION

In order to remove the defects of the conventional saccharification described above, the present inventors made investigations on enzyme-producing microorganisms, aiming at harvesting a novel glucoamylase having high thermostability in acidic pH ranges.

As a result, it has been found that a mesophilic anaerobic bacterium G-0005 separated from a medium temperature-methane fermentation slurry of organic waste fluids could produce a thermostable glucoamylase. From the fermentation broth, the novel enzyme of the present invention has been separated and as a result of extensive investigations on the saccharification of starch using this enzyme, the present invention has come to be accomplished.

When the novel thermostable glucoamylase in accordance with the present invention is employed, the starch can be saccharified without adding any calcium agent in acidic pH ranges, without neutralizing the raw starch slurry.

By the use of the liquid obtained by liquefaction using the acid-resistant and thermostable $\alpha$-amylase requiring a minimized level of calcium previously found by the present inventors (European Patent Publication No. 0184019) and by saccharifying the thus obtained liquid with the acid-resistant and thermostable glucoamylase of the present invention, the starch can be saccharified at a high rate substantially without requiring neutralization. Therefore, the burden on the desalting step subsequent to the saccharification can be greatly reduced and at the same time, the propagation of undesirable bacteria and/or fungi in the glucose production line can be prevented.

Glucose can be obtained from starch at a high rate by liquefying and saccharifying the starch in the simultaneous presence of the aforesaid $\alpha$-amylase having thermostability and acid resistance and slightly requiring calcium and the glucoamylase in accordance with the present invention, substantially without requiring either neutralizing step or cooling step.

Starch which can be used as the starting raw material of glucose in the present invention is not particularly limited but any kind of starch can be used and, known starches of potato, sweet potato, corn, tapioca, wheat, etc. is all applicable. The liquefaction may be conducted either by an enzymatic method using $\alpha$-amylase or by chemical hydrolysis using an acid.

For purposes of maintaining the thermostability of the $\alpha$-amylase in accordance with the present invention, it is unnecessary to add calcium agents, except for the case where distilled water is used as the water to be charged and amylase of a reagent grade, from which calcium ions have been completely eliminated, is used. Accordingly, when ordinary tap water is used, the desalting step for eliminating cations subsequently performed is not required substantially. The optimum reaction range of the $\alpha$-amylase is almost identical with its stable range and therefore, it is generally unnecessary to adjust the pH upon the liquefaction. For this reason, an acidic starch slurry can be reacted without adjusting its pH value.

The concentration of the raw starch varies depending upon kind and quality of the raw material and use of the liquefied matter but generally in a range of 10 to 40% conventionally used.

The reaction temperature is 100°C or below in view of the thermostability of the $\alpha$-amylase. Taking the reaction rate and thermostability of the enzyme into account, it is preferred to operate the reaction at 70 to 80°C. The quantity of the enzyme to be added varies depending upon kind and retention time of the raw starch in the reaction tank, reaction temperature, pH, etc. but, for example, in case that the treating time is fixed to 0.5 hours, approximately $10^5$ units of the enzyme are required per 1 kg of starch to hydrolyze potato starch at 80°C and pH of 4.5 to limit dextrin. The unit of the $\alpha$-amylase activity is defined by the Blue value method, which will be later described in detail together with the description of the properties of the enzyme.

The starch-liquefied product, namely, dextran which can be used in the present invention may be any one obtained by the liquefaction according to the enzymatic method or by the chemical partial decomposition treatment with an acid.

Upon the saccharification, it is generally unnecessary to adjust its pH as far as the liquefaction is operated under acidic conditions showing a pH value between 4 and 5. The concentration of the liquefied matter varies depending on kind and quality of the raw material and use but is generally in a range of 10 to 40% conventionally adopted.

The reaction is conducted generally at temperatures of 100°C or below in view of the thermostability of the glucoamylase in accordance with the present invention. Taking the reaction rate and thermostability of the enzyme into account, it is preferred to operate the reaction at 60 to 80°C.

The addition of calcium ions is not required in nature of the enzymer of the present invention.

The quantity of the enzyme to be added varies depending upon quality, concentration, retention time of the liquefied product of the raw starch in the reaction tank, reaction temperature, pH, etc. but in case that the treating time is fixed to 48 hours, approximately 120 units of the enzyme are required per 1 kg of potato starch defined in the Japanese Pharmacopoeia to saccharify limit dextrin having a saccharification rate up to 90%.

The definition of the glucoamylase activity unit will be later described in detail together with the description of the properties of this enzyme.

In case that the liquefaction and saccharification are carried out in the simultaneous presence of the $\alpha$-amylase and the glucoamylase, the reaction conditions can be appropriately chosen considering the properties of both enzymes because the temperatures, pH values, etc. of these enzymes are in the same ranges.

The $\alpha$-amylase which can be preferably used in the present invention is described in European Patent Publication No. 0184019. A bacterium belonging to the genus Clostridium (Clostridium sp. RS-0001) which can produce such $\alpha$-amylase has been deposited in the Institute of Microorganism Industrial Science and Technology, Agency of Industrial Science & Technology of Japan (Accession Number: Bikoken Kinki No. 7918 (FERM P-7918)).

Details of bacteriological properties of this bacterium are described below.

A. Morphological properties

(1) Shape of vegetative cell:

When the bacterium is cultured on an agar plate of a starch-peptone medium described below in an anaerobic atmosphere at 60°C for 2 days, the vegetative cells are a straight bacillus having a size of 0.4 to 0.8 $\times$ 2 to 5 $\mu$m. When the bacterium is cultured for 3 days or more, the vegetative cells having the aforesaid shape exist individually and linked vegetative cells are also produced. Liquid culture also gives similar results.

Composition of the Starch-Peptone Medium

| | |
|---|---|
| Soluble starch | 1.5% |
| Peptone | 0.5% |
| Yeast extract | 0.5% |
| KH$_2$PO$_4$ | 0.7% |
| Na$_2$HPO$_4$ | 0.35% |
| MgSO$_4$.7H$_2$O | 0.001% |
| Agar | 2.0% |
| Sodium thioglycolate | 0.1% |
| Tap water | |
| pH 6.4 | |

(2) Existence of spore

Formation of spores is observed in agar plate culture using the starch-peptone medium and in submerged culture.

B. Culture characteristics

(1) Shape of colony

Colonies formed in agar plate culture using the starch-peptone medium have the shape of a flat disc of which the center is slightly protuberant, and their peripheral portions are entire. No pigment is formed in the colonies. The colonies have a luster on the surface and are milk white and opaque. They are cohesive.

(2) The bacteria are grown by agar plate culture and stab culture both using a nutrient broth. The same colonies as in the case of the starch-peptone medium are formed.

### Composition of the Nutrient Agar

Meat extract     l.0%
Peptone     l.0%
Sodium chloride     0.2%
Sodium thioglycolate     0.l%
Agar     l.5%
Distilled water     pH 6.0

(3) Stab culture in the nutrient broth

The bacteria grow with generation of a gas containing hydrogen and carbon dioxide. As a result, the nutrient agar is divided in two or three places.

(4) Submerged culture in nutrient broth

The bacteria grow only in an anaerobic atmosphere and the culture solution becomes automatically turbid.

### Composition of the Nutrient Broth

Meat extract     l.0%
Sodium thioglycolate     0.l%
Distilled water
          pH 6.0

(5) Culture in broth-gelatin

The bacteria do not grow.

### Composition of the Broth-Gelatin Medium

Meat extract     l.0%
Peptone     l.0%
Sodium chloride     0.2%
Gelatin     l5%
Sodium thioglycolate     0.l%
Distilled water
          pH 6.0

(6) Culture in litmus milk

The culture is accompanied by generation of a gas, and the medium coagulates hard and is reddened by production of an acid.

### C. Physiological Properties

(l) Temperature range for growth

No range is found but the bacteria grow satisfactorily at about 60°C.

(2) pH Range for growth

The bacteria grow at pH 5 to 7. The growth is satisfactory in the vicinity of pH 5.6.

(3) Behavior toward oxygen

Obligately anaerobic.

(4) O-F Test (Hugh Laifson modified method)

The bacteria do not grow under an atmosphere of air and is negative. Under anaerobic conditions in a liquid paraffin multilayer, the bacteria grow and produce an acid so that the culture is yellowed.

Composition of the Medium

    Peptone    0.2%
    Glucose    l.0%
    Sodium chloride    0.5%
    $K_2HPO_4$    0.03%
    Sodium thioglycolate    0.l%
    Bromocresol purple    0.002%
    Agar    0.3%
    Distilled water
        pH 6.0

(5) VP Test

Negative.

(6) MR Test

Positive; the culture is reddened.

(7) Indole production

Measurement is impossible because the bacteria do not grow in aqueous peptone

(8) Hydrogen sulfide production

Negative in the case of using Kligrer medium.

(9) Hydrolysis of starch

Positive. Not only soluble starches but also granular starches such as potato starch, etc. are hydrolyzed.

(l0) Utilization of citric acid

Negative in the case of using Simmons medium.

(ll) Utilization of ammonium salts

Measurement is impossible because the bacteria do not grow in aqueous peptone.

(l2) Extracellular production of pigment

Negative.

(l3) Urease activity

Negative.

(l4) Oxidase activity

Negative.

(l5) Catalase activity

Negative.

(l6) Utilization of sugars

In the following table are shown utilization of sugars and the results of observing whether a gas generates or not by using a Durham fermentation tube. In the table, symbol + indicates the case in which the utilization of sugars and the gas generation are both observed and symbol - indicates the case in which neither utilization nor gas generation is observed.

## Table 1

| Carbon source | Utilization | Gas production |
|---|---|---|
| Glycerol | − | − |
| D-Xylose | + | + |
| D-Glucose | + | + |
| D-Fructose | + | + |
| D-Mannose | + | + |
| D-Galactose | + | + |
| L-Rhamnose | + | + |
| D-Mannitol | + | + |
| D-Sorbitol | − | − |
| Inositol | − | − |
| Trehalose | + | + |
| Lactose | + | + |
| Maltose | + | + |
| Sucrose | + | + |
| Dextrin | + | + |
| Starch | + | + |
| Inulin | − | − |
| Cellobiose | + | + |

(l7) Growth in inorganic salt medium
The bacteria do not grow.
(l8) Production of organic acids
The compositions of organic acids produced from various media are shown in Table 2.

## Table 2

| Main carbon source of media | Organic acids produced |
|---|---|
| Glucose | Acetic acid |
| | Lactic acid |
| Dextrin | Acetic acid |
| | Lactic acid |
| Starch | Lactic acid |
| Mannitol | Lactic acid |
| Sucrose | Acetic acid |
| | Lactic acid |
| Maltose | Acetic acid |
| | Lactic acid |
| Rhamnose | Acetic acid |

Composition of the Liquid Medium

| | |
|---|---|
| Carbon Source | 1.0% |
| Peptone | 1.0% |
| Sodium thioglycolate | 0.1% |
| Distilled water | |
| pH 64 | |

From these results, the bacteria were identified as bacteria belonging to the genus Clostridium according to the manual of classifying anaerobic bacteria of Holdeman.

Next, the enzymatic characteristics of the thermostable α-amylase obtained using the bacterium of the present invention are described below:

The dextrinizing ability was measured by the Blue value method (Chemical Society of Japan, Jikken Kagaku Koza, vol. 24, Biochemistry II, page 279, Maruzen Publishing Co., Ltd. (1969)). This method uses the principle that with the progress of hydrolysis of starch molecules, the level of blue color produced due to a starch-iodine complex decreases in proportion to a decrease of the molecular weight. First 2 ml of a starch solution having a concentration of 2 mg/ml and 1 ml of 0.1 M citrate buffer was shaken in a water bath at 60°C for 5 minutes. Subsequently, 1 ml of culture filtrate was added as a crude enzyme solution, and the resulting solution was reacted for 30 minutes. After the reaction is completed, 0.4 ml of the reaction solution was collected and immediately mixed with 2 ml of a 0.5 M acetic acid solution to stop the enzyme reaction. Next, 1 ml of the solution thus obtained was added to 10 ml of 1/3000 N iodine solution, and the absorbance at 680 nm was measured by means of a spectrophotometer. On the other hand, a part of the reaction solution immediately after the addition of the enzyme was collected and subjected to coloration in the same manner as described above, and the absorbance was measured. As the starch, an amylose having a polymerization degree of about 2,000 was used.

The α-amylase activity was calculated from the following equation:

$$\alpha\text{-Amylase activity (unit) } =$$

$$\frac{\text{Absorbance of reaction solution at 0 time} - \text{Absorbance of reaction solution at 30 mins.}}{\text{Absorbance of reaction solution at 0 time}} \times 10$$

(I) Action and substrate specificity

The enzyme produced by the bacteria of the present invention is liquid-type $\alpha$-amylase which hydrolyzes starches of potato, corn, sweet potato, etc.

(2) Optimum pH

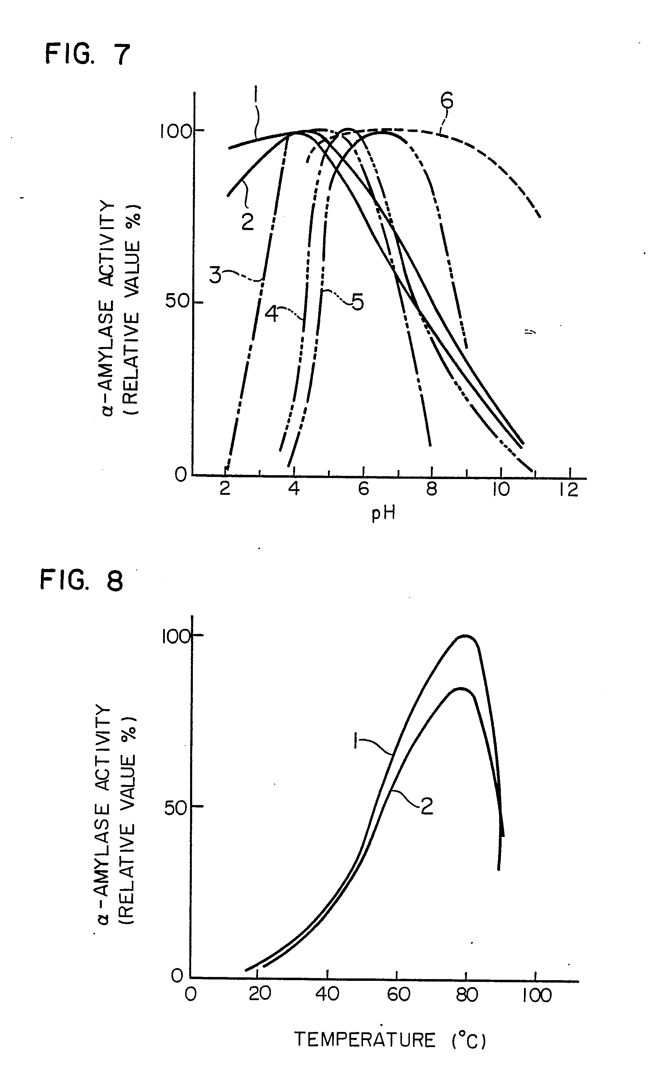

Action-pH curves of well-known typical $\alpha$-amylases are shown in Figure 7. The $\alpha$-amylase derived from Bacillus subtilis which has been reported by Ogasawara et al. (J. Biochem., 67, 65 (1970)) and is shown by curve 4, and the $\alpha$-amylase derived from Bacillus licheniformis (Japanese Patent Application Kokai (Laid-Open) No. 35083/73) which has been reported by Saito et al. and is shown by curve 6, have a suitable pH range (a pH range where each $\alpha$-amylase has 80% of the activity at the optimum pH) at pH 4 to II. The $\alpha$-amylase derived from Bacillus licheniformis which has reported by Tanaka et al. and has the highest activity on the acidic side among heretofore well-known acid $\alpha$-amylases (Japanese Patent Application Kokai (Laid-Open) No. I5I970/77, curve 3) has a suitable pH range at pH 3.5 to 6.3 and has no activity at all at pH 2.

On the other hand, $\alpha$-amylase I (curve I) and $\alpha$-amylase II (curve 2) produced by the bacterium used preferably in the present invention have an optimum pH range at 60°C at a pH of about 4, have a suitable pH range at pH 2 to 5.7 or pH 2 to 6.3, respectively, and have a high activity even on a more acidic side as compared with the conventional acid $\alpha$-amylase. That is to say, at pH 2, the conventional acid $\alpha$-amylases have no activity at all, while the $\alpha$-amylases produced by the bacterium of the present invention have a high activity of 95% or 8I%, respectively.

The following reaction system was used for the enzyme reaction:

Enzyme solution: 0.6 to I.3 $\mu$g/ml
Substrate: amylose I mg/ml
Citrate buffer : 0.025 M.

From the fact that as described above, the $\alpha$-amylases produced by the bacterium used preferably in the present invention are different in action pH range from the conventional acidic $\alpha$-amylase, it is evident that they are novel $\alpha$-amylase.

(3) pH Stability

Each of $\alpha$-amylases I and II used preferably in the present invention was incubated at pH 2, 4, 6 or 7 (0.025 M citrate buffer) at 60°C for 30 minutes. The reaction solution was diluted and then adjusted to pH 4, after which the residual activity was assayed by using amylose as substrate. As a result, both $\alpha$-amylases entirely retained their activities after the pH treatment described above. Accordingly, the present $\alpha$-amylases are characterized in that they are stable even in acidic pH ranges.

(4) Optimum temperature

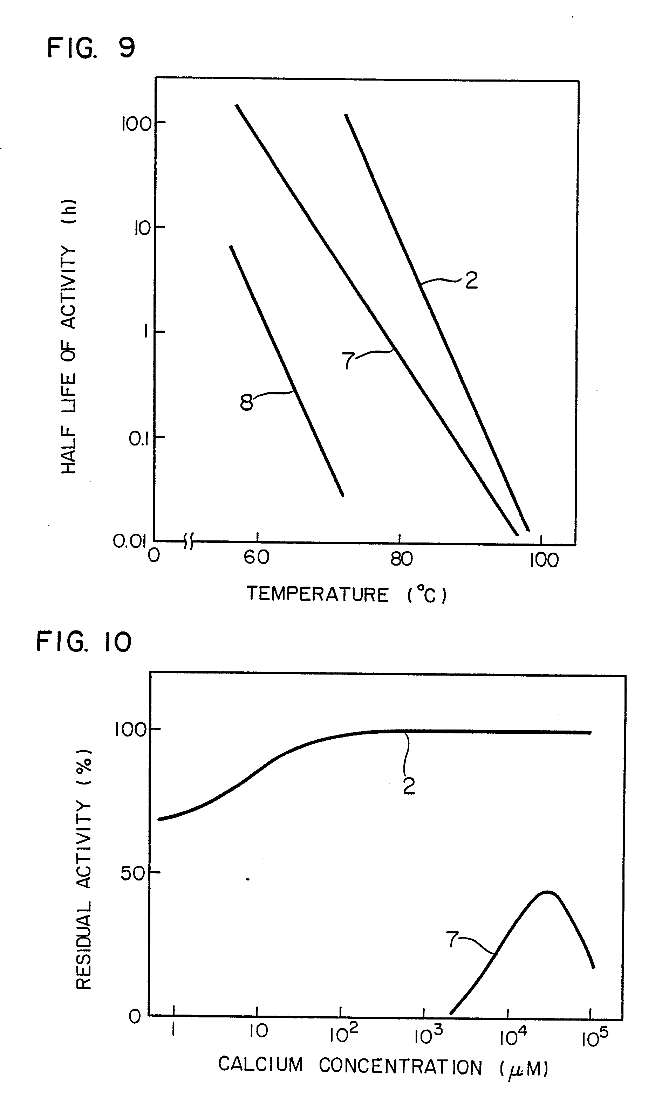

As shown in Figure 8, both of the optimum temperatures at the optimum pH of 4.0 of $\alpha$-amylases I (curve I) and II (curve 2) used preferably in the present invention are about 80°C. Their suitable temperature (a temperature at which each $\alpha$-amylase has 80% of the activity at the optimum temperature)is 65° to 87°C. For the reaction, 0.025 M citrate buffer was used.

(5) Thermostability

$\alpha$-Amylase II used preferably in the present invention was heated at 60° to 97°C at pH 6.0 in the presence of 20 $\mu$M calcium chloride, and the residual activity was measured. On the basis of the measured values, the half-life of activity at each temperature was determined. The results obtained are shown in Figure 9. The half-lives of activity (without addition of substrate) at 80°C and 90°C are 8 hours and 0.5 hour, respectively. Thus, $\alpha$-amylase II is excellent in thermostability. $\alpha$-Amylase I has a half-life of activity at 90°C of about 0.5 hours, and it is thus equal to $\alpha$-amylase II in thermostability. On the other hand, as examples of conventional $\alpha$-amylases, there were used partially purified $\alpha$-amylase preparations prepared from culture broths of an $\alpha$-amylase-producing bacterium belonging to Bacillus licheniformis and an $\alpha$-amylase-producing bacterium belonging to Bacillus subtilis, and their half-lives of activity were measured at a calcium concentration of 20 mM. The results obtained are also shown in Figure 9. The reaction was carried out by use of citrate buffer at pH 6.0 which is the optimum pH of both α-amylases. The half-life of activity at 80°C of the former α-amylase was 0.6 hour. In thermostability, the α-amylases used preferably in the present invention (curve 2) is inferior to a known thermostable α-amylase of Thermus but is by no means inferior to a thermostable α-amylase of genus Bacillus (a thermostable α-amylase derived from Bacillus licheniformis sp. curve 7).

(6) Effects of metal salts on thermostability

The effects of metal salts on the thermostability of α-amylase II used preferably in the present invention are shown in Table 3. Each of various metal salts was added to an aqueous α-amylase II solution so as to adjust the metal salt concentration to 5 mM, after which the resulting solution was heated and the activity was measured. The percentage of the activity after the heat treatment based on the activity before the heat treatment, namely, the residual activity is shown in terms of %. The heat treatment and the activity measurement are conducted under the following conditions:

Heat treatment conditionspH 6.0
Heating temperature: 80°C
Holding time : 30 minutes

## Table 3

| Metal salt added (5 mM) | Residual activity (%) |
|---|---|
| Sodium chloride | 79 |
| Potassium chloride | 54 |
| Magnesium chloride | 50 |
| Calcium chloride | 100 |
| Manganese chloride | 15 |
| Nickel chloride | <1 |
| Cobalt chloride | <1 |
| Zinc chloride | <1 |
| No addition | 60 |

The activity measurement was conducted under the following conditions after diluting each sample solution. It was confirmed that addition of each metal salt at the adding concentration had no influence on the activity measurement.

Activity measurement conditions pH 4.0 (0.025 M citrate buffer)
Activity measurement temperature: 60°C

As is obvious from Table 3, calcium ion has a protective effect for α-amylase, while ions of sodium, potassium, magnesium, nickel, cobalt, zinc and manganese make the thermostability lowered. It was also confirmed that this α-amylase lost its thermostability in the presence of 0.5 μm of EDTA.

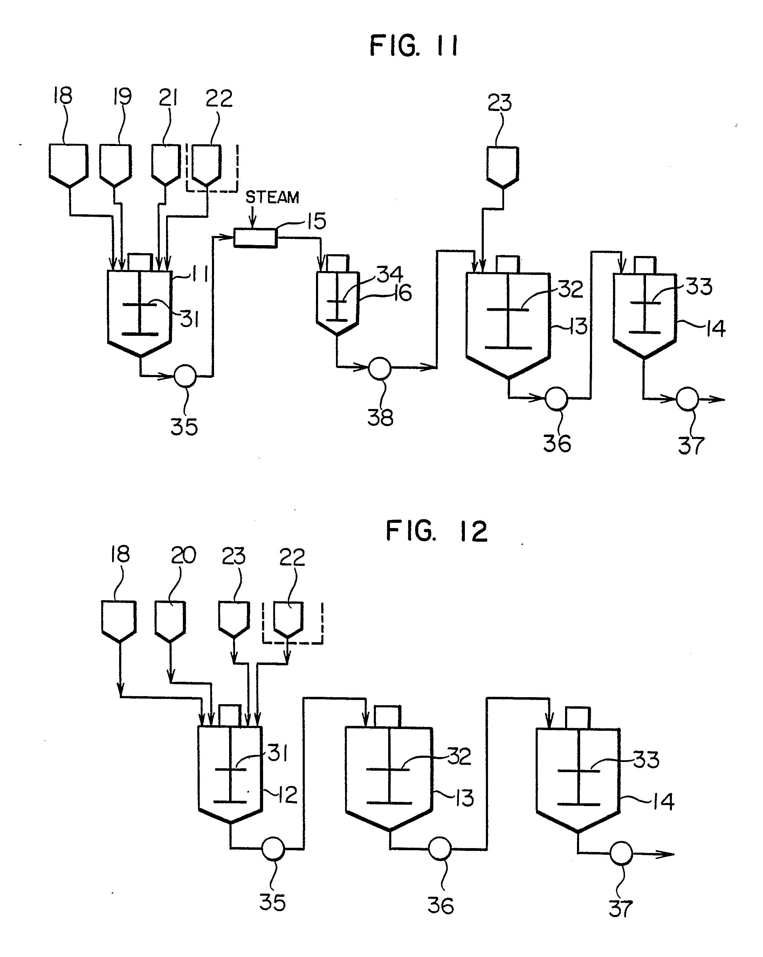

The concentration of calcium required of the α-amylase is 100 μM (4 ppm) as shown in curve 2 in Figure 10, and the α-amylase is sufficiently stabilized at the calcium concentration in tap water. Further, this enzyme retains 65% of the activity even at a calcium concentration of 1 μM or less. α-Amylase I has a calcium requirement equal to that of $\alpha$-amylase II.

On the other hand, an $\alpha$-amylase partially purified from an $\alpha$-amylase-producing bacterium belonging to Bacillus licheniformis requires 30 mM of calcium ions as shown in curve 7 in Figure 10. The heat treatment was carried out by heating at 80°C for 30 minutes at pH 6 for both enzymes, and the activity measurement was conducted at 60°C at the respective optimum pHs.

On the other hand, thermostable $\alpha$-amylase of Bacillus subtilis has a calcium concentration of 3 to 10 mM (Japanese Patent Application KOKAI (Laid-Open) Nos. 44690/76 and 34117/83).

Accordingly, the $\alpha$-amylases used preferably in the present invention have a much smaller calcium requirement as compared with the known thermostable $\alpha$-amylases.

(7) Purification method

Since the purification method is described in detail in Examples, it is briefly described here.

An $\alpha$-amylase-producing bacterium was inoculated into a liquid medium containing starch, peptone and yeast extract, and cultured under anaerobic conditions at 60°C for 1 to 3 days. Cells and other insoluble substances are removed from the culture broth by centrifugation and the like to obtain a so-called culture filtrate. Subsequently, concentration of $\alpha$-amylases used in the present invention and removal of impurities are conducted by properly applying well-known methods such as molecular sieve membrane filtration, ion exchange chromatography, gel filtration chromatography, salting out and the like to the culture filtrate.

(8) Molecular weight

Although the molecular weights of the $\alpha$-amylases used preferably in the present invention have not yet been confirmed, they are estimated to be 20,000 or more from their behaviors in molecular sieve membrane filtration.

As described above, the thermostable $\alpha$-amylases used preferably in the present invention are markedly different particularly in action pH and calcium requirement from thermostable enzymes produced by conventional aerobic bacteria.

Next, the novel glucoamylase having acid resistance and thermostability used in the present invention and a bacterium capable of producing the glucoamylase will be described below.

A bacterium belonging to the Clostridium sp. G-0005 which can produce the $\alpha$-amylase used in the present invention has been deposited in the Institute of Microorganism Industrial Science and Technology, Agency of Industrial Science & Technology of Japan (Accession Number: Bikoken Kinki No. 8737 (FERM P-8737)). Details of bacteriological properties of this bacterium are described below. Percentages are all by weight unless otherwise indicated.

A. Morphological properties

(1) Shape of vegetative cell:

When the bacterium is cultured on an agar plate of a starch-peptone medium described below in an anaerobic atmosphere at 60°C for 2 days, the vegetative cells are a straight bacillus having a size of 0.3 to 0.5 × 2 to 4 μm. When the bacterium is cultured for 3 days or more, the vegetative cells having the aforesaid shape exist individually and linked vegetative cells are also produced. Liquid culture also gives similar results.

Composition of the Dextrin-Peptone Medium

Dextrin      1.5%
Peptone      0.5%
Yeast extract      0.5%
$KH_2PO_4$      0.7%
$Na_2HPO_4$      0.2%
$MgSO_4.7H_2O$      0.001%
Agar      2.0%
Sodium thioglycolate      0.1%
Tap wter
      pH 6.0

(2) Existence of mobility

No mobility is observed.

(3) Existence of spore

Formation of spores is observed in agar plate culture using the starch-peptone medium. The spores are formed at the intracellular end of the vegetative cells and are spheres having a diameter of approximately 0.5 μm.

(4) Gram staining

Negative.

B. Culture characteristics

(l) Shape of colony

Colonies formed in agar plate culture using the starch-peptone medium have the shape of a flat disc of which the center is slilghtly protuberant, and their peripheral portions are irregular. No pigment was produced in the colonies. The colonies are milk white and translucent.

(2) Agar plate culture in the nutrient medium

The bacteria do not grow.

Composition of the Nutrient Agar Medium

    Meat extract     l.0%
    Peptone     l.0%
    Sodium chloride     0.2%
    Sodium thioglycolate     0.l%
    Agar     2.0%
    Distilled water
          pH 6.0

(3) Slant culture in the nutrient agar

The bacteria do not grow.

(4) Stab culture in the nutrient agar

The bacteria grow slightly along with the stab line. No pigment is produced.

(5) Submerged culture in the nutrient broth

The bacteria do not grow.

Composition of the Nutrient Broth

    Meat extract     l.0%
    Peptone     l.0%
    Sodium chloride     0.2%
    Sodium thioglycolate     0.0l%
    Distilled water
          pH 6.0

(6) Culture in broth-gelatin

The bacteria do not grow. Gelatin is not liquefied.

Composition of the Broth-Gelatin Medium

    Meat extract     l.0%
    Peptone     l.0%
    Sodium chloride     0.2% .

Gelatin    l5.0%
Sodoium thioglycolate    0.l%
Distilled water
      pH 6.0

(7) Culture in litmus milk

The culture produces an acid and the medium coagulates hard. Generation of a gas is observed.

C. Physiological Properties

(l) Temperature range for growth

The bacteria grow at 49 to 64°C but do not grow at 46°C and 69°C. The bacteria grow satisfactorily in the vicinity of 57 to 6l°C.

(2) pH Range for growth

The bacteria grow at pH 4.8 to 7.5. The growth is satisfactory in the vicinity of pH 6.0

(3) Behavior toward oxygen

Obligately anaerobic.

(4) O-F Test (Hugh Laifson Method)

Negative. The bacteria grow under an atmosphere of air and under anaerobic conditions in a liquid paraffin multilayer, both accompanied by generation of a gas. The bacteria grow and produce an acid so that the culture is yellowed. In the culture in the atmosphere of air, the bacteria grow from about l cm of the interface with the gaseous phase toward the bottom.

Composition of the Medium

Peptone    0.2%
Glucose    l.0%
Sodium chloride    0.5%
$K_2HPO_4$    0.03%
Bromocresol purple    0.002%
Agar    0.3%
Distilled water
      pH 6.0

(5) Reduction of nitrates

Negative.

(6) VP Test

Negative.

(7) MR Test

Positive; the culture is reddened.

(8) Indole production

Measurement is impossible because the bacteria do not grow in aqueous peptone.

(9) Hydrogen sulfide production

Negative in the case of using Kligrer medium.

(l0) Hydrolysis of starch

Positive.

(ll) Utilization of citric acid

Negative in the case of using Simmons medium.

(l2) Utilization of ammonium salts

Measurement is impossible because the bacteria do not grow in aqueous peptone.

(l3) Extracellular production of pigment

Negative.

(l4) Urease activity

Negative.

(l5) Oxidase activity

Negative.

14

(l6) Catalase activity
Negative.
(l7) Utilization of sugars

In Table 4 are shown utilization of sugars and the results of observing whether a gas generates or not by using a Durham fermentation tube. In the table, symbol + indicates the case in which the utilization of sugars and the gas generation are both observed and symbol -indicates the case in which neither utilization nor gas generation is observed.

## Table 4

| Carbon source | Utilization | Gas production |
|---|---|---|
| D-Xylose | + | + |
| D-Arabinose | - | - |
| D-Glucose | + | + |
| D-Fructose | + | + |
| D-Mannose | + | + |
| D-Galactose | + | + |
| L-Rhamnose | - | - |
| D-Mannitol | - | - |
| D-Sorbitol | - | - |
| Inositol | - | - |
| Trehalose | - | - |
| Lactose | + | + |
| Maltose | + | + |
| Sucrose | + | + |
| Cellobiose | + | + |
| L-Sorbose | - | - |
| Salicin | + | + |
| Inulin | - | - |
| Dextrin | + | + |
| Starch | + | + |
| Cellulose | - | - |
| Glycerol | - | - |

### Composition of the Submerged Medium for Sugar Utilization

```
Carbon source (sugar)      1.0%
Peptone      1.0%
Sodium chloride      0.2%
Sodium thioglycolate      0.1%
Distilled water
          pH 6.0
```

(18) Growth in the inorganic salt medium

The bacteria do not grow.

From the foregoing results, the bacteria were identified as bacteria belonging to the genus Clostridium based on the Bergey's Manual of Determinative Bacteriology, 8th Edition.

Next, the enzymatic characteristics of the thermostable α-glucoamylase of the present invention are described below:

The aglucoamylase activity was measured as follows.

Soluble starch (manufactured by Wako Junyaku Co., Ltd., for biochemical use) was used as a substrate for the measurement of the glucoamylase activity. Firstly, 0.5 ml of 5% soluble starch solution, 0.5 ml of 0.1M acetate-sodium acetate buffer having pH 4.5, 1 ml of pure water and 0.5 ml of an enzyme solution were mixed and, an enzymatic reaction was conducted at 60°C for 30 minutes. Then, a quantity of glucose in the reaction mixture was determined using a glucose analyzer (manufactured by Yellow Springs Instrument Company, USA: measured by the glucose oxidase method). One glucoamylase activity unit was defined as the amount of enzyme required to produce 1 μmol of glucose in 1 hour under the assay conditions described above.

### (1) Preparation

The preparation will later be described in the examples in details and is thus briefly described herein.

The bacterium G-0005 belonging to the genus Clostridium in accordance with the present invention is inoculated on a liquid medium containing dextrin, peptone and yeast extract followed by incubation at 60°C for 1 to 3 days under anaerobic conditions. The culture solution is centrifuged to remove cells and other insoluble matters and a so-called culture filtrate is obtained. Subsequently, the culture filtrate is appropriately subjected to known means such as molecular sieve membrane filtration, salting out, ion exchange chromatography, gel filtration chromatography, etc. to concentrate the glucoamylase and at the same time, remove the impurities.

The carbon source for the liquid medium in liquid culture is not limited to the dextrin described above but there may also be used soluble starch, potato starch, corn starch, sweet potato starch, blackstrap molasses, etc. Further other nutrient components are not limited to those described above but corn steep liquor, various amino acids, vitamins, various salts, etc. may also be used singly or in combination.

### (2) Action and Substrate Specificity

The enzyme is a glucoamylase which hydrolyzes starch of potato, corn, sweet potato, etc., and soluble starch, dextrin, maltose or the like obtained by hydrolysis ofsuch starch. In the case of the maltose substrate, a rate of producing glucose is approximately 1/2 that of the soluble starch substrate.

### (3) Optimum pH

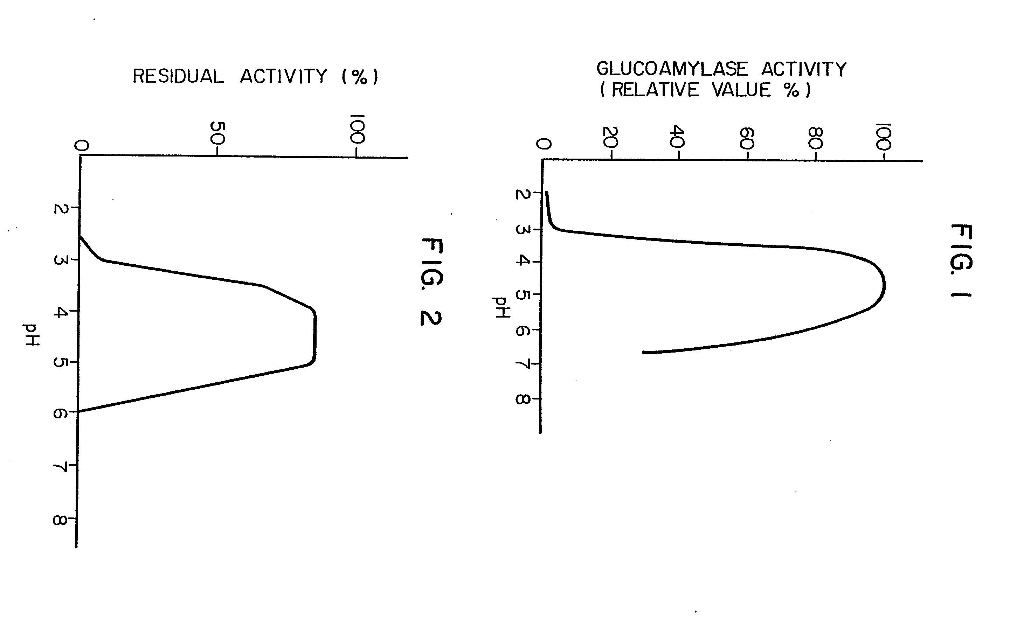

A pH curve of the enzyme acted at 60°C is shown in Figure 1. The optimum pH of the enzyme at 60°C is in a range between 4 and 5 and a preferred range (which is defined as a pH range showing 80% of the activity at the optimum pH) is between 3.8 and 5.7. As the pH buffer used upon the reaction, 0.05M buffer of potassium chloride-hydrochloride (pH 2.0), glycine-hydrochloride (pH 2.5 to 3.5) or β,β'-dimethylglutarate-tris(hydroxymethylamino)methane-2-amino-2-methyl-1-1,3-propanediol (hereafter simply referred to as "GTA") (pH 3.5 to 9) was employed.

## (4) pH Stability

The glucoamylase of the present invention was incubated at 70°C for 30 minutes at respective pH values of 2, 3, 4, 4.5, 5, 6, 7 and 9 (using 0.05M acetate-hydrochloride buffer and 0.05M GTA buffer). Thereafter, the reaction solution was diluted. After adjusting pH to 4.5, the residual activity was measured using soluble starch as a substrate. The results are shown in Figure 2. The glucoamylase of the present invention completely retained its activity in a pH range of 4.5 to 5.0. Therefore, it was noted that the glucoamylase was an enzyme having excellent stability in acidic pH ranges.

## (5) Optimum temperature

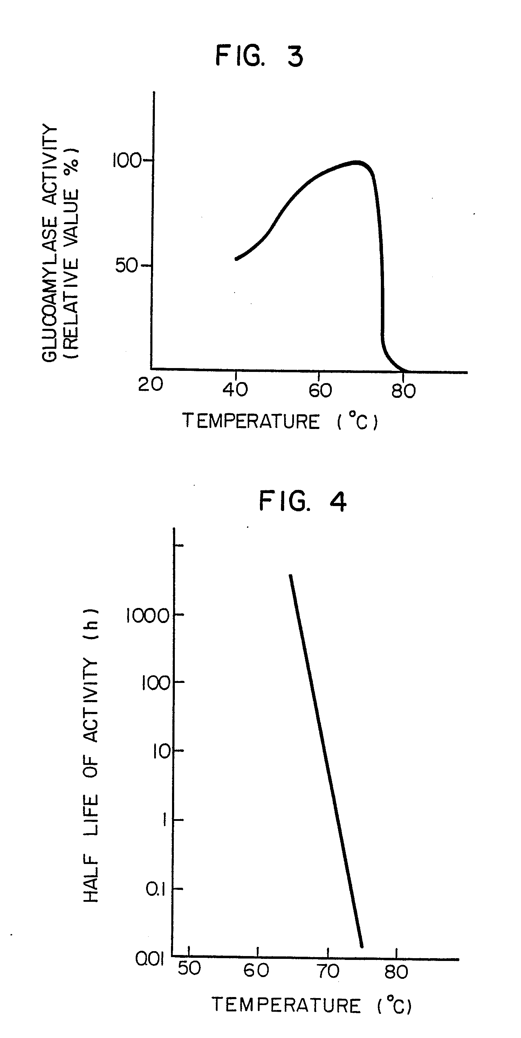

The glucoamylase activity of the present invention was measured at temperatures of 40, 50, 60, 65, 70 and 75°C, respectively, at pH 4.5. The results shown in Figure 3 were obtained. From the results, the optimum temperature lies in the vicinity of 70°C. A preferred temperature range (which is defined as a temperature range showing 80% of the activity at the optimum temperature) is between 53 and 73°C.

## Table 5

| Enzyme | Half Life of Activity (min.) | | | | |
| | pH 4.0 | pH 4.3 | pH 4.5 | pH 5.0 | pH 5.0 |
|---|---|---|---|---|---|
| Glucoamylase of this invention | 340 | 340 | 360 | 330 | 20 |
| Glucoamylase of Clostridium thermoamylticum a) | — | <5 | — | .68 | 215 |
| Glucoamylase of Thermomyces lanuginosus b) | — | — | — | — | 30 |
| Glucoamylase of Talaromyces duponti c) | — | 14 | — | 24 | 11 |

a)   Japanese Patent Application KOKAI (Laid-Open) No. 54680/85

b)   Biochem. J., 193, pages 379-387 (1981)

c)   U.S. Patent 4,247,637

## (6) Influence of metal salts on the glucoamylase activity

The influence of metal salts on the activity of the glucoamylase of the present invention is shown in Table 6. In the measurement of the glucoamylase activity, various metal salts were added in a concentration of 5 mM. The activity to an activity when no metal salt was added is expressed in terms of %.

The results obtained with the cases of adding an ammonium salt and EDTA are also shown in Table 6. It is noted that ions of magnesium, calcium and potassium activate the glucoamylase activity but ions of nickel and iron inhibit the glucoamylase activity.

17

## Table 6

| Metal Salt Added | Concentration | Relative Activity |
|---|---|---|
| Zinc chloride | 5 mM | 100 |
| Nickel chloride | " | 66 |
| Manganese chloride | " | . 102 |
| Cobalt chloride | " | 95 |
| Ferric chloride | " | <1 |
| Magnesium chloride | " | 110 |
| Calcium chloride | " | 110 |
| Potassium chloride | 50 mM | 110 |
| Ammonium chloride | 50 mM | 102 |
| EDTA | 0.5 μM | 100 |
| None | - | 100 |

Conditions for the measurement of the activity:
pH 5.0 (0.IM citrate-sodium citrate buffer);
Temperature for the activity measurement: 60°C.

(7) Thermostability

The glucoamylase of the present invention was subjected to a heat treatment at 60 to 80°C at pH 4.5 in the absence of a substrate and a part of the treated solution was collected every definite time (20 and 40 seconds, I, 2, 4, I0, 20 and 40 minutes, I, 2, 4, 6, 8 and I6 hours and I, 2, 4, 7, I0, I5 and 20 days). The glucoamylase activity in each solution was measured at 60°C and pH 4.5. Based on the activity values, the half life of activity was determined at each temperature. The results are shown in Figure 4. The half lives of activities at 70°C and 75°C are 6 hours and I minute, respectively, in the absence of the substrate. The glucoamylase has thermostability to a higher degree than in known glucoamylases. On the other hand, the half life of activity at 70°C was determined at respective pH values of 4.0, 4.3, 4.5, 5.0 and 6.0 in the absence of the substrate and the results are shown in Table 5. The results reveal that the glucoamylase has a superior thermostability to glucoamylases produced by Clostridium thermoamylalyticum, Thermomyces lanuginosus and Talaromyces dupont, particularly in the acidic pH range of 4 to 5.

(8) Influence of metal salts on thermostability

The influence of metal salts on the thermostability of the glucoamylase of the present invention is shown in Table 7. Various metal salts were added to an aqueous solution of glucoamylase (dissolved in 0.05M acetate-sodium acetate buffer, pH 4.5) in a concentration of 5 mM. After a heat treatment at 70°C for an hour, the glucoamylase activity was measured at pH 4.5 and 60°C. The activity of glucoamylase after the heat treatment to that prior to the heat treatment, namely, the residual activity, is expressed in terms of %.

From Table 7, it is noted that nickel ions, manganese ions and magnesium ions had a protective effect but cobalt ions and calcium ions had no protective effect; zinc ions markedly reduced the thermostability.

## Table 7

| Metal Salt Added (5 mM) | Residual Activity (%) |
|---|---|
| Zinc chloride | <1 |
| Nickel chloride | 112 |
| Manganese chloride | 126 |
| Cobalt chloride | 91 |
| Magnesium chloride | 103 |
| Calcium chloride | 93 |
| None | 94 |

(9) Molecular weight

The molecular weight of the glucoamylase of the present invention is determined as $3.8 \times 10^4$ from the elution pattern given by gel filtration chromatography (using Sephadex G-I00 manufactured by Pharmacia Co., Ltd., Sweden).

As is clear from the foregoing, the novel thermostable glucoamylase of the present invention is markedly different from conventional thermostable enzymes produced by anaerobic bacteria particularly in its thermostability in the acidic pH range of 4 to 5.

To produce glucose or isomerized glucose, the starch as a raw material is firstly liquefied by α-amylase followed by saccharification by glucoamylase. In the liquefaction, the raw starch is charged in a concentration as high as 20 to 40% so that the resulting starch slurry has an acidic pH. For this reason, in the case of using conventional α-amylase and glucoamylases, the slurry must be neutralized because the action pH is in neutral ranges. To the contrary, the thermostable and acid-resistant α-amylase and glucoamylase used in the present invention possess high activity even in acidic pH ranges and therefore, the liquefaction and saccharification can be carried out in the acidic state without requiring any neutralization in the liquefaction (in this case, even the pH adjustment is not necessarily required) so that the burden on the desulting step subsequent to the reaction can be greatly reduced.

Further the novel glucoamylase used in the present invention can maintain its activity even at high temperatures of 80°C or more and therefore, can be used as it is, without cooling, even after the liquefaction at a high temperature of 80°C.

The glucose production plant used for the liquefaction step and saccharification step of the present invention described above in detail comprises:

a liquefaction tank comprising a starch slurry making tank comprising a starch storage tank, a water storage tank and an α-amylase storage tank and, a slurry heating tank; and,

a saccharification tank comprising a glucoamylase storage tank, which is connected with the liquefaction tank.

The saccharification tank is preferably a thermostat. The starch slurry making tank may be additionally equipped with a pH adjusting chemical storage tank.

In the production plant of the present invention, the liquefaction tank may not be substantially equipped with any device for neutralizing the starch. Further the saccharification tank may not be substantially equipped with any device for neutralizing the dextrin. Furthermore, the production plant may not substantially contain any device for supplying metal ions to stabilize the α-amylsae of glucoamylase.

Hereafter the production plant of the present invention is described with reference to the drawings.

Figure II shows an example of the glucose production device in accordance with the present invention. In this example, starch is used as a raw material. In starch slurry making tank II equipped with a stirrer and a pH adjusting mechanism, water and starch are charged from water storage tank I8 and starch storage tank I9, respectively, and stirred to make a starch slurry. It is desired that the water used herein contain about

100 μM calcium salts. When industrial water, tap water for drinking or the like is generally used as raw water, it is unnecessary to add calcium salts to the water. Raw starch is not particularly limited but potato starch, corn starch, sweet potato starch, etc. can be employed. It is desired that the concentration of starch slurry be between 10 and 40%. It is also desired that the pH be previously measured when the starch slurry is heated and gelatinized and a pH adjusting chemical be added from pH adjuster storage tank 22 to adjust the pH after gelatinization to 4 to 5. In many cases, however, when the starch slurry is in a gelatinized form, a pH value appears generally between 4 and 5 and therefore, the adjusting chemical rarely needs to be added. α-Amylase is added to the starch slurry, the pH of which has been corrected, from α-amylase storage tank 21 followed by through stirring. Subsequently, the starch slurry is transferred to heater 15 via feeding pump 35. In the slurry heater, steam is directly injected into the starch slurry. The starch slurry is instantaneously heated to 100 to 105°C and immediately transferred to liquefaction tank 16. In liquefaction tank 16, the slurry is maintained at 70 to 100°C for 10 to 60 minutes, during which gelatinization of the starch and liquefaction by α-amylase are performed, thereby rapidly reducing the viscosity of the liquid. After completion of the liquefaction, the liquefied liquid is transferred to saccharification tank 13 through feeding pump 38. The saccharification tank 13 is equipped with a stirrer, a heating mechanism and a pH measurement device. Glucoamylase is fed into the liquefied liquid from glucoamylase storage tank 23, whereby enzyme reaction is carried out at 70 to 80°C for 10 to 50 hours. Namely, the dextrin formed by the liquefaction is hydrolyzed to glucose. After completion of the saccharification, the saccharified liquid is transferred to saccharification liquid storage tank 14 via feeding pump 36 and stored there until it is ready to be transferred to the subsequent purification step. Upon transfer to the purification step, it is fed under pressure via feeding pump 37.

Figure 12 is a flow chart showing an example of the glucose production device according to the present invention. In this example, dextrin is used as a raw material for the saccharification. The raw dextrin is fed into dextrin solution making tank 12 from dextrin storage tank 20. Water is added thereto from water storage tank 18 and the mixture is stirred by stirrer 31 to prepare a dextrin solution having a solid content of 10 to 80 wt%. Subsequently, the pH of dextrin solution is measured and, if necessary, a pH adjusting chemical is added from pH adjusting chemical storage tank 22 to adjust the pH to 4 to 5. Then $1 \times 10^4$ to $5 \times 10^5$ units/g of solid of glucoamylase are added from the glucoamylase storage tank followed by stirring and mixing them. Thereafter, the mixture is transferred to saccharification tank 13 through feeding pump 35. The saccharification tank 13 is equipped with stirrer 32, a heating device and a pH measurement device. The dextrin solutoin is heated to 70 to 80°C and maintained for 10 to 50 hours, during which dextrin is hydrolyzed by glucoamylase to glucse. After completion of the saccharification, the saccharified liquid is transferrd to saccharified liquid storage tank 14 via feeding pump 36 and stored there. Upon transfer to the purification step, it is fed via feeding pump 37.

According to the present invention, the reaction can be carried out at high temperatures in such an acidic state as it is, without neutralizing the raw starch slurry. For this reason, the reaction can proceed at a high rate and requires no addition of any neutralizing agent or calcium salts so that the burden on the desalting step of the saccharified solution can be greatly reduced.

Hereafter, the present invention will be described in more detail, by referring to the examples below.

Example 1 Preparation of glucoamylase

(i) Preparation of culture filtrate containing glucoamylase:

32 Kilograms of liquid media (pH 6.0) containing 1.5% of dextrin, 0.5% of polypeptone, 0.5% of yeast extract, 0.7% of potassium dihydrogenphosphate, 0.2% of disodium hydrogenphosphate, 0.001% of magnesium sulfate heptahydrate, 0.1% of sodium thioglycolate and tap water were divided into ten equal parts, and 3.15 kg of the media was charged in each of ten culture tanks having a capacity of 5 liters and sterilized at 120°C for 15 minutes. To the media in each tank was added 0.35 kg of a cell suspension of bacterium G-0005 belonging to the genus Clostridium which had been anaerobically cultured in the same media as described above. Subsequently, a water-sealing trap was attached to the gas outlet, and the gas phase portion in each fermentation tank was thoroughly replaced with argon gas, after which the bacterium was cultured under anaerobic conditions. The pH of the culture broth was automatically adjusted to 6.0 and the temperature was also automatically adjusted to 60°C. After the bacteria were cultured for 21 hours, the culture broths were combined and centrifuged at 6,000 r.p.m. to remove the cells. The recovered supernatants were filtered under pressure through glass filter papers (manufactured by Toyo Kagaku Sangyo Co., Ltd., GA-100 and GC-50) and membrane filters (manufactreud by Toyo Kagaku Sangyo Co.,

Ltd.: pore sizes of I μm and 0.45 μm), whereby the cells and other solid substances were removed to give the culture filtrate. From the culture filtrate was taken up I0 ml. After dialysis against pure water for 24 hours, its glucoamylase activity was measured and 0.05 units of glucoamylase was present per I g of the culture filtrate.

(ii) Purification by means of molecualr sieve membrane:

Next, 29 kg of the culture filtrate described above was filtered through a hollow fiber type molecular sieve membrane (cut-off molecular weight: I0,000, manufactured by Amicon Co., USA, HIP I0-20) under pressure and concentrated to 2 kg.

(iii) Purification by means of salting out by ammonium sulfate:

The aforesaid concentrate was subjected to salting out using ammonium sulfate and the solid substance which did not precipitate at the saturation (a rate to the saturated concentration of ammonium sulfate, expressed in terms of %) of 20% but precipitated at the saturation of 55% was centrifuged and recovered by centrifugation at I4,000 r.p.m. Subsequently, the solid substance was washed with teh ammonium sulfate solution having a saturation of 55% and recovered. Then, the solid substance was dissolved in 0.05 M Tris-hydrochloride buffer (pH 7.5) to make the volume 240 g.

(iv) Purification by means of dialysis:

Using I0 liters of the buffer described above, dialysis for 24 hours was repeated twice. Thereafter, the solid substances in the dialyzed solution were filtered through glass filter paper (manufactured by Toyo Filter Ppaer Co., Ltd., GC-50) to remove it. A clear dialysate was 3I0 g.

(v) Purification by means of ion exchange chromatography:

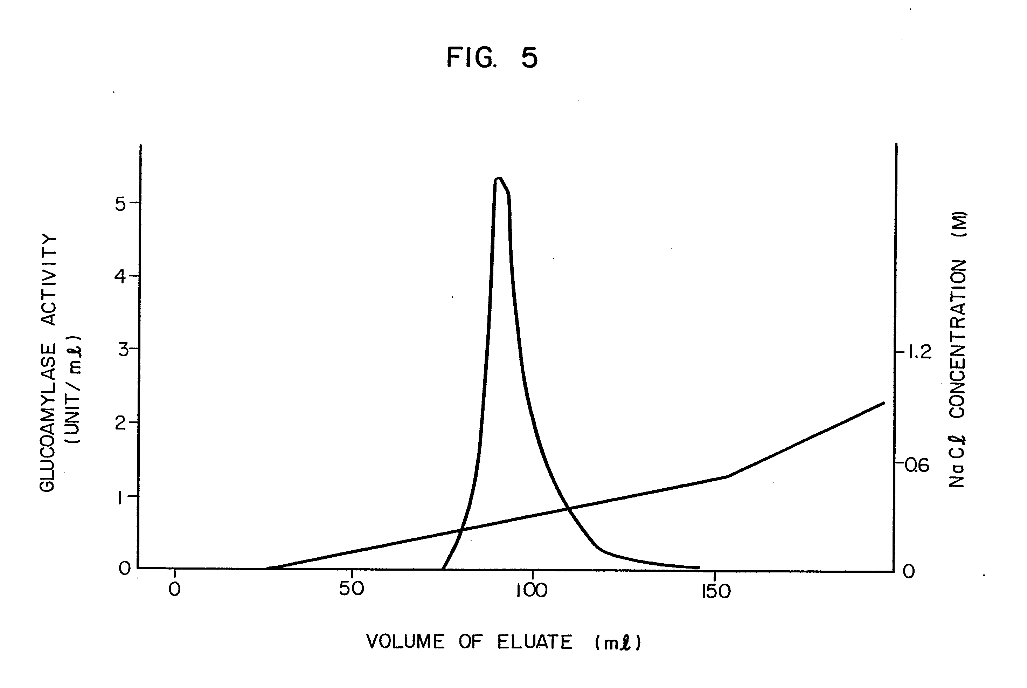

Next, the dialysate described above was purified by means of an ion exchange chromatography (column size of ⌀44 × 500 mm) on diethylaminoethylated crosslinked agarose gel (manufactured by Pharmacia co., Ltd., DEAE Sepharose CL-6B). The aforesaid dialysate was charged into a gel column, which had been equilibrated with 0.05 M Tris-hydrochloride buffer, and then washed. Subsequently, elution was carried out using a linear gradient increasing the sodium chloride concentration in the buffer. The elution pattern of the glucoamylsae is shown in Figure 5. The peak having the glucoamylase activity was observed at an elution position corresponding to a sodium chloride concentration of 0.25 M. As the glucoamylase fraction was recovered 360 g. After adding I0 kg of pure water thereto, the mixture was filtered through a molecular sieve membrane (cut-off molecular weight: I0,000, manufactured by Amicon Co., USA, PM-I0) under pressure, desalted and concentrated to give I60 g of a crude glucoamylase solution. The solution had a glucoamylase activity or 3.3 units/g.

(vi) Purification by means of gel filtration:

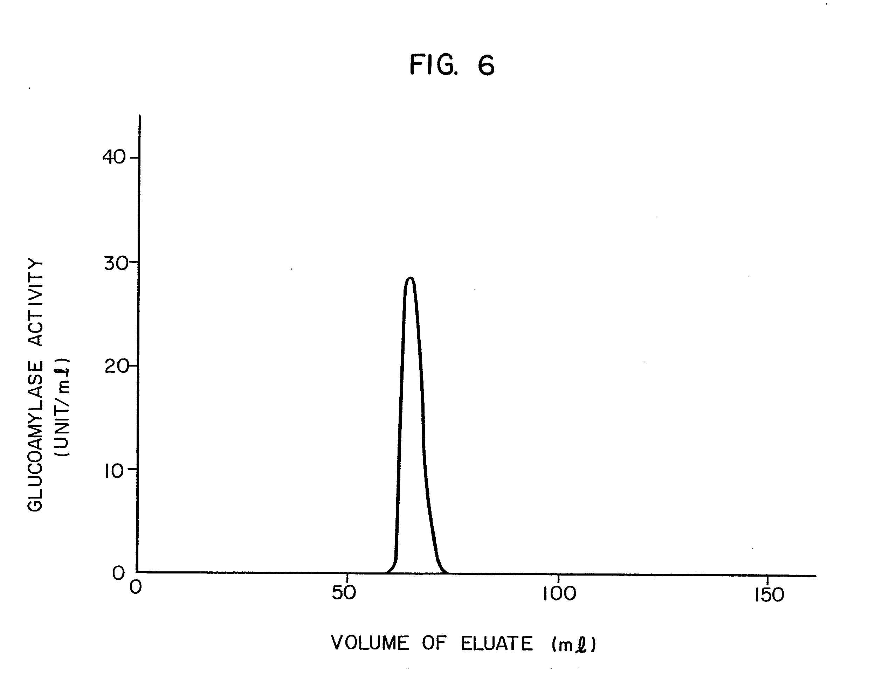

Next, the crude glucoamylase described above was purified by gel filtration. Firstly, I00 g of the glucoamylase solution described above was freeze-dried under reduced pressure of 40 Torr and dissolved in 4 ml of 0.05 M citrate-dicitrate buffer (pH 4.5). Solid substances were removed by centrifugation at 4,000 r.p.m. Next, I ml of the aforesaid glucoamylsae solution was charged into a chromatography column (⌀I5 × 900 mm) packed with crosslinked dextran gel, which had been equilibrated with the buffer described above, and then eluted with the same buffer. The results are shown in Figure 6. The peak having the glucoamylsae activity was observed at an elution position of 65 ml. The gel filtration described above was carried out also with the remaining crude glucoamylase to obtain 40 g of a purified-glucoamylase fraction. The fraction had a glucoamylase activity of I2 units/g.

21

From the above purification operations, the specific activity was improved by about 171 times the specific activity of the culture filtrate. The recovery of activity was 23% based on the culture filtrate. In Table 8 are shown the specific activity, yield and recovery of activity from the culture filtrate in each step.

## Table 8

| Item<br>Preparation | Specific<br>Activity<br>(unit/g) | Yield<br>(g) | Recovery of<br>Activity<br>(%) |
|---|---|---|---|
| Culture filtrate | 0.07 | 29,000 | 100 |
| Concentrate obtained using a molecular sieve membrane | 0.98 | 2,000 | 96 |
| Glucoamylase fraction obtained by precipitation with ammonium sulfate | 5.6 | 240 | 66 |
| Dialysate | 3.5 | 310 | 53 |
| Crude glucoamylase by ion exhange chromatography | 3.3 | 160 | 26 |
| Glucoamylase purified by gel filtration | 12 | 40 | 23 |

Example 2 Purification of glucoamylsae from the cell-homogenized supernatant:

In Example I, 400 g of the solid substance containing the cells was recovered from the culture broth by centrifugation. Then, 600 g of physiological saline was added to the solid substance to make a slurry and 1000 g of the cell slurry was obtained. Subsequently, 40 g was taken out of the cell slurry and set in a pressure cell for French press (manufactured by Otake Seisakusho). After a pressure of 1600 kg/cm² was applied by a hydraulic press, the slurry was injected from a narrow opening into the air at a rate of 5 ml/min. to homogenize the cells. The above procedure was performed also with the remaining cell slurry to give 1000 g of the cell-homogenized slurry.

Next, solid substances were removed from the cell slurry by centrifugation at 16,000 r.p.m. and the supernatant was filtered under pressure through glass filter papers (manufactured by Toyo Kagaku Sangyo Co., Ltd., GA-100 and GC-50) and membrane filters (manufactured by Toyo Kagaku Sangyo Co., Ltd.: pore sizes of I $\mu$m and 0.45 $\mu$m) to give 800 ml of the cell-homogenized supernatant.

Subsequently, the cell-homogenized supernatant was subjected to a heat treatment at 70°C for 5 minutes to denaturate the protein liable to cause thermal denaturation. After cooling to 5°C or below, solid substances were removed by centrifugation at 8,000 r.p.m. The thus heat-treated supernatant was 760 ml.

Next, the respective purification procedures of precipitation with ammonium sulfate, dialysis, an ion exchange chromatography and gel filtration were carried out in a manner similar to Example I. In Table 9 are shown the specific activity, yield and recovery of activity based on the cell-homogenized supernatant in each step.

22

## Table 9

| Item<br>Preparation | Specific<br>Activity<br>(unit/g) | Yield<br>(g) | Recovery of<br>Activity<br>(%) |
|---|---|---|---|
| Cell-homogenized supernatant | 4.75 | 800 | 100 |
| Heat-treated supernatant | 4.60 | 760 | 92 |
| Glucoamylase fraction obtained by precipitation with ammonium sulfate | 30.6 | 80 | 64 |
| Dialysate | 20.9 | 110 | 60 |
| Crude glucoamylase obtained by ion exchange chromatography | 8.8 | 160 | 37 |
| Glucoamylase purified obtained by gel filtration | 61 | 20 | 32 |

Example 3 Purification of glucoamylase

20 Kilograms of liquid media (pH 6.4) containing 2.3% of corn starch, 0.2% of polypetrone, 0.2% of ammonium sulfate, 0.2% of potassium dihydrogenphosphate, 0.002% of magnesium sulfate heptahydrate, 0.1% of cystein and tap water were charged in a fermenter having a capacity of 50 liters and sterilized at 120°C for 20 minutes by blowing steam into the fermenter and a jacket portion mounted on the outer wall of the fermenter. Afrter cooling the inside of the fermenter to 60°C, sterilized water was added to make the amount of the media 32 kg. Next, 3.5 kg of a cell suspension of bacterium G-0005 belonging to the genus Clostridium which had been prepared using the same media in a manner similar to Example I was added to the media. Subsequently, a water-sealing trap was attached to the gas outlet, and the gas phase portion in each fermenter was thoroughly replaced with nitrogen gas, after which the bacterium was cultured under anaerobic conditions at 60°C and pH 6.5. After the bacteria were cultured for 40 hours, the culture broth was immediately centrifuged using a continuous centrifuging machine to remove solid substances. The supernatant was 33.5 kg and has a glucoamylase activity of 0.09 units/g. Subsequently, 2 liters of the supernatant described above were taken up in a flask for a rotary evaporator and distilled under reduced pressure using the rotary evaporator, while immersing in a water bath of 60°C. In order to prevent foaming at the beginning of the distillation, the pressure reduction was initiated after I ml of n-octyl alcohol was added. A fresh supernatant was replenished from the outside into the flask correspondingly to the quantity of the decreased supernatnat in the flask and, the distillation was continued under reduced pressure. Whenever the foaming in the flask became vigorous, n-octyl alcohol was added at any time. By the distillation under reduced pressure, 2.3 ℓ of the concentrate was obtained.

Next, the concentrate described above was subjected to a heat treatment at 70°C for 5 minutes. After immediately cooling to 50°C or below, the solid substances precipitated by the heat treatment were removed by centrifugation at 7,000 r.p.m. to give 2.I liters of the heat-treated supernatant.

The heat-treated supernatant was subjected to salting out using ammonium sulfate and a fraction showing the saturation of 20 to 55% was recovered. Subsequently, the fraction was washed with ammonium sulfate solution having a saturation of 55%. Then, the solid substance was dissolved in distilled water to give 520 g of a crude glucoamylase solution.

23

Next, 200 g of corn starch was added to the crude glucoamylase solution. After thoroughly mixing, the mixture was centrifuged to separate corn starch and the supernatant was recovered. Further the corn starch was washed with 200 g of distilled water and the washing liquid was recovered. The washing liquid was combined with the supernatant described above to give 490 g of a starch adsorption-treated solution. Then, the solution was filtered through glass filter papers (manufactured by Toyo Filter Paper Co., Ltd.; GA-I00 and GC-50) under pressure to obtain 480 g of a crude glucoamylase solution. The solution had a glucoamylsae activity of 5.3 units/g. The specific activity was improved by 59 times that of the supernatant and the recovery of activity was 84%.

Example 4 <u>Production of glucose from potato starch using α-amylase and glucoamylase</u>

(i) <u>Preparation of α-amaylase:</u>

15.75 Kilograms of liquid media (pH 6.5) containing I.5% of soluble starch, 0.5% of polypeptone, 0.5% of yeast extract, 0.7% of potassium dihydrogenphosphate, 0.35% of disodium hydrogenphosphate, 0.0I% of magnesium sulfate heptahydrate, 0.I% of sodium thioglycolate and tap water were divided into five equal parts, and 3.I5 kg of the media was charged in each of five fermenters having a capacity of 5 liters and sterilized at I20°C for 20 minutes. To the media in each fermenter was added 350 g of a cell suspension of α-amylase-producing Clostridium sp. RS-000I (FERM P-79I8; European Patent Publication No. 0I840I9) belonging to the genus Clostridium which had been separated by the present inventors and anaerobically cultured in the same media. Subsequently, a water-sealing trap was attached to the gas outlet, and the gas phase portion in each fermenter was thoroughly replaced with argon gas, after which the bacterium was cultured under anaerobic conditions. The pH of the culture broth was automatically adjusted to 6.0 and the temperature was also automatically adjusted to 60°C. After the bacteria were cultured for 22 hours, the culture broths were combined and centrifuged at 6,000 r.p.m. to remove the cells. The supernatant had a specific activity of 60 units/g.

Next, after I4.6 kg of the supernatant described above was cooled to 2°C, 300 g of corn starch and 300 g of celite (manufactured by Wako Junyaku Co., Ltd.) were added to contact with the supernatant for I0 minutes with stirring. Subsequently, the corn starch and celite were recovered and the system was further washed with 3 liters of pure water chilled to 2°C. The aforesaid corn starch and celite were dispersed in 0.05 M Tris-HCl buffer (pH 7.5) containing 5 mM calcium chloride, which had been previously warmed at 65°C. After maintaining on a water bath at 65°C for 5 minutes, the dispersion was subjected to solid-liquid separation by filtration under reduced pressure. Further the solid substance was washed with I liter of the same buffer (65°C). The filtrate and washing liquid were combined to give 4 liters. After cooling to 2°C, the aforesaid filtrate was filtered through a molecular sieve membrane (cut-off molecular weight: 20,000) to concentrate. Solid substances were removed by further filtration to give 400 ml of the concentrate. The concentrate had an α-amylase activity of $1.5 \times 10^3$ units/ml.

Next, the concentrate described above was purified by means of an ion exchange chromatography (column size: oö50 $\times$ 2I0 mm) using diethylaminoethylated crosslinked agarose gel (DEAE Sepharose CL-6B, manufactured by Pharmacia Co., Ltd.). Firstly, the aforesaid concentrate was dialyzed twice with 0.05 M Tris-HCl buffer (pH 7.5). Insoluble substances were filtered and charged into a gel column, which had been equilibrated with the same buffer, and then washed. Subsequently, development was carried out while increasing the sodium chloride concentration in the buffer with a linear gradient. As a result, two peaks having the glucoamylase activity was observed at elution positions corresponding to sodium chloride concentrations of 0.04 M and 0.08 M. The activity layer of α-amylase I eluted at the position corresponding to 0.04 M was about 30% of the total adsorbed activity, while that of α-amylase II eluted at the position corresponding to 0.08 M was about 60%. Both active fractions were dialyzed to pure water and freeze dried to obtain α-amylase I and α-amylase II. The recoveries of activities based on the centrifugation supernatant of the culture broth were I7% and 32%, respectively.

(ii) <u>Preparation of glucoamylase:</u>

Next, I20 kg of liquid media (pH 6.0) containing I.5% of dextrin, 0.5% of polypeptone, 0.5% of yeast extract, 0.7% of potassium dihydrogenphosphate, 0.2% of disodium hydrogenphosphate, 0.00I% of magnesium sulfate heptahydrate, 0.I% of sodium thioglycolate and tap water were divided into ten equal parts, and I2 kg of the media was charged in each of ten fermenters having a capacity of 20 liters and sterilized at

24

120°C for 15 minutes. To the media in each fermenter was added 0.5 kg of a cell suspension of bacterium G-0005 (FERM P-8737) belonging to the genus Clostridium which was the glucoamylase-producing bacterium in accordance with the present invention. Subsequently, a water-sealing trap was attached to the gas outlet, and the gas phase portion in each fermenter was thoroughly replaced with nitrogen gas, after which the bacterium was cultured at 60°C and pH of 6.0 for 2 days under anaerobic conditions. The culture broths were centrifuged at 6,000 r.p.m. for 10 minutes to remove the cells. The recovered supernatant was filtered under pressure through glass filter paper to give the solid particle-free supernatant. The supernatant contains 0.05 units/ml of glucoamylase.

Next, 115 kg of the supernatant described above was filtered under reduced pressure through a molecular sieve membrane (cut-off molecular weight: 20,000), further washed and concentrated to give 12 kg of the concentrate. The concentrate was subjected to salting out with ammonium sulfate having a saturation of 55% and the precipitate was recovered by centrifugation. The precipitate was dissolved in 0.005 M Tris-HCl buffer (pH 7.5) to give 980 g of the solution. The solution was further dialyzed for 24 hours.

Then, purification was conducted by an ion exchange chromatography (⌀44 × 500 mm) using DEAE-agarose gel to recover 360 ml of a fraction having the glucoamylase activity. The fraction was filtered through a molecular sieve membrane to give 160 mg of an enzyme solution showing 30 units/ml. The solution was freeze dried and subjected to gel filtration on a column of crosslinked dextran gel to obtain 40 g of the active fraction (92 units/ml).

(iii) Production of glucose from potato starch:

Next, 2000 units of the aforesaid thermostable α-amylase were added to a mixture of 10 g of potato starch defined in the Japanese Pharmacopoeia and 90 ml of hot water of 90°C. The mixture was reacted at 80°C for 3 hours, without neutralizing the reaction system by the addition of an alkali or without adding metal ions to the mixture, to convert into limit dextrin.

Subsequently, 100 units of the aforesaid glucoamylase were added to the liquefaction-treated solution described above. The mixture was reacted at 70°C and pH 4.5 for 3 hours, without neutralizing the reaction system by the addition of an alkali or without adding metal ions to the mixture, to perform saccharification.

As a result, a saccharified solution showing DE (Dextrose Equivalent Value) of 95.8 was obtained:

$$DE = \frac{\text{Direct Reducing Sugar (calculated as glucose)}}{\text{Solid Content}} \times 100$$

Example 5 Production of glucose from corn starch using α-amylaes and glucoamylase

In an autoclave having a capacity of 0.3 liters equipped with a stirrer were charged 10 g of corn starch, 90 g of tap water and 1000 units of the α-amylase prepared in Example 4. While stirring, steam having a gauge pressure of 2 kg/cm² was blown into the gas phsae portion of the autoclave for 10 seconds while opening an exhaust valve. Then, the exhaust valve was closed and steam was blown into the corn starch slurry, whereby the inner pressure was adjusted to 1.1 kg/cm², which was maintained for an hour. Subsequently, the liquefied product was withdrawn from the autoclave. After cooling to 80°C, 1000 units of α-amylase was added and the mixture was reacted for 2 hours to prepare a liquefied matter.

To the liquefied matter were added 1000 units of the glucoamylase prepared in Example 4. The mixture was reacted at 70°C for 3 hours. In both liquefaction step and saccharification step described above, neither neutralization by the addition of an alkali nor supplementation of metal ions was performed.

As a result, a saccharified liquid having DE of 93.2 was obtained. The pH of the reaction solution was 4.8 after the saccharification.

Example 6 Production of glucose from sweet potato starch using α-amylase and glucoamylase

Liquefaction and saccharification were carried out with 10 g of sweet potato starch in a manner similar to Example 4 to give a saccharified liquid showing DE of 96.3.

## 0 255 124

Example 7 <u>Production of glucose from potato starch in the co-existence of α-amylase and glucoamylase</u>

To l0 g of potato starch defined in the Japanese Pharmacopoeia was added 90 ml of tap water followed by heating at 70°C. Subsequently, 2000 units of the α-amylase and l000 units of the glucoamylase prepared in Example 4 were added to the mixture. The mixture was reacted at 70°C for 5 hours. After completion of the reaction, the reaction mixture showed pH 4.8 and DE of 92.8. Neither neutralization nor addition of metal ions were performed.

While the invention has been described in detail and with reference to specific embodiments thereof, it will be apparent to one skilled in the art that various chagnes and modifications can be made therein without departing from the spirit and scope thereof.

**Claims**

l. A thermostable glucoamylase having the following physicochemical properties:
(l) Action optimum pH: 4 to 5
(2) Action optimum temperature: about 70°C
(3) Stable pH: 4.5 to 5.0
(4) A half life of enzyme activity at pH 4.5 and a temperature of 70°C is at least 6 hours.
(5) A half life of enzyme activity at pH 4.0 and a temperature of 70°C is at least 5.5 hours.
(6) Molecular weight: $3.8 \times 10^4$.

2. A thermostable glucoamylase according to Claim l, obtainable from a thermophilic and anaerobic bacterium belonging to the genus Clostridium.

3. A thermostable glucoamylase according to Claim 2, wherein said thermophilic and anaerobic bacterium is Clostridium sp. G-0005 (Accession No. FERM P-8737).

4. A thermophilic and anaerobic bacterium belonging to the genus Clostridium capable of producing a thermostable glucoamylase having the following physicochemical properties:
(l) Action optimum pH: 4 to 5
(2) Action optimum temperature: about 70°C
(3) Stable pH: 4.5 to 5.0
(4) A half life of enzyme activity at pH 4.5 and a temperature of 70°C is at least 6 hours.
(5) A half life of enzyme activity at pH 4.0 and a temperature of 70°C is at least 5.5 hours.
(6) Molecular weight: $3.8 \times 10^4$.

5. A thermophilic and anaerobic bacterium according to Claim 4, wherein said thermophilic and anaerobic bacterium is Clostridium sp. G-0005 (Accession No. FERM P-8737).

6. A process for production of a glucose by saccharifying dextrin using a glucoamylase, which comprises reacting the raw dextrin at a temperature of at least 70°C in an acidic pH range substantially without adding metal ions to saccharify the dextrin.

7. A process for production of a glucose according to Claim 6, wherein said glucoamylase is a thermostable glucoamylase having acid resistance and thermostability which has an action optimum pH between 4 and 5 and a half life of activity at pH 4.5 of at least 3 hours at 70°C and at least l20 hours at 65°C and requires no calcium ions to exhibit the thermostability

8. A process for production of a glucose according to Claim 6, which uses a thermostable glucoamylase according to any of the Claims 1 to 3.

9 . A process for production of a glucose by liquefying a starch using an α-amylase and saccharifying the produced dextrin using a glucoamylase, which comprises reacting the raw starch at a temperature of at least 70°C in an acidic pH range substantially without adding metal ions to perform the liquefaction and saccharification of the raw starch.

10. A process for production of a glucose according to Claim 9, wherein said α-amylase is a thermostable α-amylase having acid resistance and thermostability which has an action optimum pH between 2 and 5.5, a half life of activity at pH 4.5 and 80°C being at least 80 hours and exhibits the thermostability in the presence of at most l0 μM calcium ions.

11. A process for production of a glucose according to Claim 10, wherein said thermostable α-amylase is a thermostable α-amylase produced from a thermophilic and anaerobic bacterium belonging to the genus Clostridium.

12. A process for production of a glucose according to Claim 10, wherein said thermostable α-amylase is a thermostable α-amylase produced from Clostridium sp. RS-000l (Accession No. FERM P-79l8).

13. A process for production of a glucose according to any of the Claims 9 to 12, wherein said glucoamylase is a thermostable glucoamylase having acid resistance and thermostability which has an action optimum pH between 4 and 5 and a half life of activity at pH 4.5 of at least 3 hours at 70°C and at least 120 hours at 65°C and requires no calcium ions to exhibit the thermostability.

14. A process for production of a glucose according to any of the Claims 9 to 12, wherein said glucoamylase is a thermostable glucoamylase according to any of the Claims 1 to 3.

15. The process for production of a glucose according to any of the Claims 9 to 14, characterized by simultaneously adding the α-amylase and the glucoamylase to perform the liquefaction and saccharification of the raw starch.

16. A plant for producing a glucose by liquefaction of a starch using an α-amylase followed by saccharification using a glucoamylase, which comprises:

a liquefaction tank comprising a starch slurry making tank comprising a starch storage tank, a water storage tank and an α-amylase storage tank, and a slurry heater; and,

a saccharification tank comprising a glucoamylase storage tank, having connected with said liquefaction tank.

17. A plant for production of glucose according to Claim 16, wherein said saccharification tank is a thermostat.

18. A plant for production of glucose according to Claim 16, wherein said starch slurry making tank is additionaly equipped with a pH adjusting chemical storage tank.

19. A plant for production of glucose according to Claim 16, wherein said liquefaction tank is not substantially equipped with a device for neutralizing the starch.

20. A plant for production of glucose according to Claim 16, wherein said saccharification tank is not substantially equipped with a device for neutralizing the dextrin.

21. A plant for production of glucose according to Claim 16, which is not substantially equipped with a device for suppling metal ions to stabilize said α-amylase.

22. A plant for production of glucose according to Claim 16, which is not substantially equipped with a device for suppling metal ions to stabilize said glucoamylase.

23. A plant for saccharification of dextrin using a glucoamylase, which is not substantially equipped with a device for neutralizing the dextrin, is not substantially equipped with a device for suppling metal ions to stabilize said glucoamylase and is equipped with a glucoamylase storage tank.

B-2/06

# FIG. 1

# FIG. 2

B-2/06

# FIG. 3

GLUCOAMYLASE ACTIVITY (RELATIVE VALUE %)

TEMPERATURE (°C)

# FIG. 4

HALF LIFE OF ACTIVITY (h)

TEMPERATURE (°C)

FIG. 5

FIG. 6

B-2106

## FIG. 7

## FIG. 8

FIG. 9

FIG. 10

B-2106

# FIG. 11

# FIG. 12